# EUROPÄISCHE PATENTANMELDUNG

(11) **EP 4 285 958 A1**
(43) Veröffentlichungstag der Anmeldung: **06.12.2023**
(21) Anmeldenummer: 23176033.1
(22) Anmeldetag: 30.05.2023
(51) Int. Cl.: A61M 5/168, A61M 39/22, A61M 5/14, A61M 39/00

(54) **MEDIZINISCHE FLUIDSTEUERVORRICHTUNG**

(30) Priorität: 01.06.2022 DE 102022205578
(71) Anmelder: B. Braun Melsungen AG, 34212 Melsungen (DE)
(72) Erfinder: Kunschak, Ralf, 6130 Willisau (CH)
(74) Vertreter: Patentanwälte Ruff, Wilhelm, Beier, Dauster & Partner mbB

(57) **Zusammenfassung**

Medizinische Fluidsteuervorrichtung (1) für ein medizinisches Fluidleitungssystem, aufweisend ein Gehäuse (2) mit einem Einlass (3), einem Auslass (4) und mit einer Aufnahmeaussparung (5), einen Steuerkörper (6), der wenigstens zwischen einer ersten Schaltstellung (P1) und einer zweiten Schaltstellung (P2) relativ zu dem Gehäuse (2) um eine Drehachse (Z) drehbeweglich in der Aufnahmeaussparung (5) aufgenommen ist, einen ersten Fluidkanal (7) mit einem ersten Strömungswiderstand und einen zweiten Fluidkanal (8) mit einem zweiten Strömungswiderstand, wobei der erste Fluidkanal (7) und der zweite Fluidkanal (8) jeweils an dem Steuerkörper (6) und/oder an dem Gehäuse (2) ausgebildet sind, wobei in der ersten Schaltstellung (P1) der Ein- und Auslass (3, 4) mittels des ersten Fluidkanals (7) fluidleitend miteinander verbunden sind, wobei in der zweiten Schaltstellung (P2) der Ein- und Auslass (3, 4) mittels des ersten Fluidkanals (7) und des zweiten Fluidkanals (8) fluidleitend miteinander verbunden sind, wobei der erste Fluidkanal (7) und der zweite Fluidkanal (8) fluidleitend parallelgeschaltet sind.

## Beschreibung

Die Erfindung betrifft eine medizinische Fluidsteuervorrichtung für ein medizinisches Fluidleitungssystem.

Medizinische Fluidsteuervorrichtungen sind in der Medizintechnik seit geraumer Zeit bekannt. Sie finden gewöhnlich bei der Infusionstherapie, der Transfusion, der Dialyse oder ähnlichen Therapiefeldern Verwendung. Typischerweise erlauben sie, Flüssigkeitswege eines medizinischen Fluidleitungssystems, welches eine derartige medizinische Fluidsteuervorrichtung umfasst, entsprechend dem gewünschten Therapieverlauf anzupassen. Die medizinische Fluidsteuervorrichtung dient dabei üblicherweise zur Steuerung eines Volumenstroms an Fluid, welches im Betrieb des medizinische Fluidleitungssystem durch dasselbe und durch die medizinische Fluidsteuervorrichtung geführt wird. Je nach Behandlung und gegebenenfalls verwendetem Wirkstoff sowie dessen Konzentration in dem Fluid sind unterschiedlich große Volumenströme für eine erfolgreiche Therapie erforderlich. Eine Abweichung von einem im Hinblick auf eine bestimmte Therapie vorbestimmten Volumenstrom kann zu einer Unter- oder Überdosierung des Wirkstoffs mit entsprechend negativen Folgen für den Patienten führen.

Gewöhnlich wird zum vorstehend genannten Zweck eine medizinische Fluidsteuervorrichtung in Form einer sogenannten Rollenklemme oder eines Präzisionsflussstellers eingesetzt. Solchen herkömmlichen Fluidsteuervorrichtungen ist es gemein, dass sie ein variables Einstellen des Volumenstroms an durch die medizinische Flusssteuervorrichtung geführtem Fluid erlauben, und zwar durch zumeist reversible Manipulation eines im Betrieb durchströmten Leitungsquerschnitts.

Medizinische Fluidsteuervorrichtungen nach der Art einer Rollenklemme sind bekannt aus der DE 19 930 172 A1, der CN 201524310 U und der CN 202802370 U. Eine medizinische Fluidsteuervorrichtungen des Typs Präzisionsflusssteller geht hervor aus der DE 10 2019 203 846 A1.

Aufgabe der Erfindung ist es, eine medizinische Fluidsteuervorrichtung bereitzustellen, welche gegenüber herkömmlichen medizinischen Fluidsteuervorrichtungen verbesserte Eigenschaften aufweist und insbesondere eine Einstellung des Volumenstroms auf einen vorbestimmten Wert vereinfacht.

Diese Aufgabe wird durch das Bereitstellen einer medizinischen Fluidsteuervorrichtung mit den Merkmalen des Anspruchs 1 gelöst. Bevorzugte Ausgestaltungen sind Gegenstand der abhängigen Patentansprüche.

Die erfindungsgemäße medizinische Fluidsteuervorrichtung ist zur Verwendung in einem medizinischen Fluidleitungssystem vorgesehen und weist ein Gehäuse mit einem Einlass, einem Auslass und mit einer Aufnahmeaussparung sowie einen Steuerkörper auf, welcher wenigstens zwischen einer ersten Schaltstellung und einer zweiten Schaltstellung relativ zu dem Gehäuse um eine Drehachse beweglich, insbesondere wenigstens teilweise, in der Aufnahmeaussparung aufgenommen ist, und wenigstens einen ersten Fluidkanal mit einem ersten Strömungswiderstand und einen zweiten Fluidkanal mit einem zweiten Strömungswiderstand, wobei der erste Fluidkanal und der zweite Fluidkanal jeweils an dem Steuerkörper und/oder an dem Gehäuse ausgebildet sind, und wobei in der ersten Schaltstellung der Einlass und der Auslass mittels des ersten Fluidkanals fluidleitend miteinander verbunden sind, und wobei in der zweiten Schaltstellung der Einlass und der Auslass mittels des zweiten Fluidkanals fluidleitend miteinander verbunden sind. Somit sind in Abhängigkeit der Schaltstellung des relativ zum Gehäuse drehbeweglichen Steuerkörpers der Einlass und der Auslass mittels des ersten sowie - alternativ oder zusätzlich - mittels des zweiten Fluidkanals fluidleitend miteinander verbunden. Mit anderen Worten lässt sich durch die Wahl der Schaltstellung des Steuerkörpers festlegen, welche Fluidkanäle den Ein- und den Auslass fluidleitend miteinander verbinden. Ein zwischen dem Einlass und dem Auslass herrschender Gesamtströmungswiderstand, von dem bei gegebener Druckdifferenz zwischen Ein- und Auslass ein die Fluidsteuervorrichtung im Betrieb durchströmender Volumenstrom an Fluid abhängt, ergibt sich somit aus den jeweiligen Strömungswiderständen der in der gewählten Schaltstellung durchströmten Fluidkanäle. Der Gesamtströmungswiderstand lässt sich also abhängig von der Schaltstellung des Steuerkörpers zwischen wenigstens zwei vorbestimmten Werten umschalten. Folglich ermöglicht die erfindungsgemäße medizinische Fluidsteuervorrichtung je nach Schaltstellung des Steuerkörpers unterschiedliche, vordefinierte und auf den jeweiligen Anwendungszweck vorabgestimmte Volumenströme an durch die medizinische Fluidsteuervorrichtung geführtem Fluid, insbesondere ohne das Erfordernis einer zeitaufwändigen und fehleranfälligen manuellen Feinjustierung. Die durch Drehverstellen des Steuerkörpers relativ zum Gehäuse erfolgende Auswahl der Schaltstellung erweist sich ferner als besonders robust gegen ein versehentliches Verstellen aus der gewählten Schaltstellung. Eine Gefahr eines solches versehentlichen Verstellens kann durch eine etwaige Patientenbewegung oder bei Pflegetätigkeiten am Patienten gegeben sein. Darüber hinaus ist es praktisch ausgeschlossen, dass sich der Steuerkörper im Betrieb der Fluidsteuervorrichtung durch den anliegenden Fluiddruck oder Schwerkraftwirkung in unerwünschter Weise löst und die gewählte Schaltstellung selbsttätig verlässt. Der in der gewählten Schaltstellung erreichbare Volumenstrom lässt sich somit besonders einfach und zuverlässig konstant halten.

In Ausgestaltung der Erfindung ist eine dritte Schaltstellung vorhanden, in welcher der Einlass und der Auslass mittels des Steuerkörpers fluiddicht voneinander getrennt sind. Vorteilhaft kann somit durch Wahl der dritten Schaltstellung eine Fluidströmung an durch die medizinische Fluidsteuervorrichtung geführtem Fluid unterbunden werden, wenn eine solche Fluidströmung unerwünscht ist. Entsprechend kann der Steuerkörper vor dem Beginn einer Behandlung eines Patienten mittels der medizinischen Fluidsteuervorrichtung oder nach Abschluss der Behandlung des Patienten in seine dritte Schaltstellung bewegt werden, um eine unkontrollierte Abgabe des Fluids zu vermeiden. Auch eine zeitweise Unterbrechung der Behandlung des Patienten lässt sich mittels der dritten Schaltstellung realisieren.

In weiterer Ausgestaltung der Erfindung sind in der zweiten Schaltstellung der Einlass und der Auslass mittels sowohl des ersten Fluidkanals als auch des zweiten Fluidkanals fluidleitend miteinander verbunden, wobei der erste Fluidkanal und der zweite Fluidkanal fluidleitend parallelgeschaltet sind. Somit lässt sich in der zweiten Schaltstellung ein besonders geringer Gesamtströmungswiderstand zwischen Ein- und Auslass erreichen. Entsprechend eignet sich die zweite Schaltstellung besonders für einen Spülvorgang zum Reinigen der medizinischen Fluidsteuervorrichtung oder eines dieselbe umfassenden medizinischen Fluidleitungssystems - insbesondere in Vorbereitung auf eine anstehende Behandlung eines Patienten. Auch falls die Therapie des Patienten die Abgabe einer besonders großen Fluidmenge in kurzer Zeit erfordert, kann die zweite Schaltstellung gewählt werden, um dem Patienten einen entsprechend großen Volumenstrom an Fluid über die medizinische Fluidsteuervorrichtung zuzuführen.

In weiterer Ausgestaltung der Erfindung sind der erste Fluidkanal und der zweite Fluidkanal zwischen jeweils einer ersten Mündungsöffnung und einer gemeinsamen zweiten Mündungsöffnung erstreckt, wobei in der ersten Schaltstellung der Einlass in die erste Mündungsöffnung des ersten Fluidkanals und von dort über den ersten Fluidkanal und die gemeinsame zweite Mündungsöffnung in den Auslass mündet, und wobei in der zweiten Schaltstellung der Einlass in die gemeinsame zweite Mündungsöffnung und von dort über die beiden Fluidkanäle und die jeweilige erste Mündungsöffnung in den Auslass mündet. Eine derartige medizinische Fluidsteuervorrichtung baut besonders kompakt. Zudem erlaubt es die vorstehend beschriebene Ausbildung der Fluidkanäle in besonders einfacher Weise, den ersten und den zweiten Schaltzustand zu realisieren.

In weiterer Ausgestaltung der Erfindung ist in der ersten Schaltstellung die erste Mündungsöffnung des zweiten Fluidkanals fluiddicht verschlossen. Somit ist der Gesamtströmungswiderstand zwischen Ein- und Auslass in der ersten Schaltstellung ausschließlich durch den Strömungswiderstand des ersten Fluidkanals bestimmt, so dass sich besonders präzise ein konstanter Volumenstrom erreichen lässt. Insbesondere lässt sich so ein besonders kleiner Volumenstrom präzise einstellen. Eine zeitaufwändige und vor allem fehleranfällige Feinjustierung des für eine bestimmte Therapie vorab festgelegten Volumenstroms an Fluid, welches dem Patienten mittels der Fluidsteuervorrichtung zuzuführen ist, wird somit obsolet.

In weiterer Ausgestaltung der Erfindung ist ein zwischen dem Ein- und dem Auslass vorhandener und durchströmbarer Gesamt-Strömungswiderstand in der ersten Schaltstellung größer als in der zweiten Schaltstellung. Somit kann in der ersten Schaltstellung ein bei der Therapie eines Patienten wünschenswerter geringer Volumenstrom realisiert werden, wohingegen in der zweiten Schaltstellung ein größerer Volumenstrom erreichbar ist. Folglich eignet sich die zweite Schaltstellung besonders für einen Spülvorgang der medizinischen Fluidsteuervorrichtung. Denn bei einem solchen Spülvorgang ist ein besonders großer Volumenstrom wünschenswert.

In weiterer Ausgestaltung der Erfindung umfasst der Steuerkörper einen ersten Durchgang, durch welchen der erste Fluidkanal gebildet ist. Der erste Durchgang kann als Bohrung ausgeführt sein. Vorzugsweise weist der erste Durchgang einen runden, insbesondere kreisförmigen, oder einen eckigen Querschnitt auf. Eine medizinische Fluidsteuervorrichtung mit einem derartigen Steuerkörper ist besonders einfach herstellbar. Insbesondere ist eine uneinheitliche Querschnittsgestaltung möglich, so dass sich der Querschnitt über eine Erstreckung des ersten Durchgangs ändert.

In weiterer Ausgestaltung der Erfindung umfasst der Steuerkörper eine erste Nut, die an einer Außenumfangsfläche des Steuerkörpers abschnittsweise ausgenommen ist und durch welche der erste Fluidkanal gebildet ist. Dies erfordert eine besonders geringe Schwächung eines Materials des Steuerkörpers.

In weiterer Ausgestaltung der Erfindung umfasst das Gehäuse eine erste Nut, die an einer Innenumfangsfläche der Aufnahmeaussparung abschnittsweise ausgenommen ist, wobei der erste Fluidkanal durch die erste Nut gebildet ist. Somit ergibt sich für das Material des Steuerkörpers aus der Führung des ersten Fluidkanals keinerlei Schwächung. Dies erweist sich als besonders vorteilhaft, wenn der Steuerkörper als, insbesondere dünnwandiger, Hohlkörper realisiert wird.

In weiterer Ausgestaltung der Erfindung umfasst der Steuerkörper eine erste Nut, die an seiner Außenumfangsfläche abschnittsweise ausgenommen ist, wobei das Gehäuse eine erste Gehäusenut umfasst, die an der Innenumfangsfläche der Aufnahmeaussparung abschnittsweise ausgenommen ist, wobei der erste Fluidkanal durch die erste Nut und durch die erste Gehäusenut gebildet ist. Die erste Gehäusenut kann die erste Nut zur gemeinsamen Führung des zweiten Fluidkanals komplettieren. Eine derart ausgestaltete medizinische Fluidsteuervorrichtung baut besonders kompakt. Zudem lassen sich auf diese Weise geometrisch besonders komplexe Verläufe des ersten Fluidkanals umsetzen.

In weiterer Ausgestaltung der Erfindung umfasst der Steuerkörper einen zweiten Durchgang, durch welchen der zweite Fluidkanal gebildet ist. Der zweite Durchgang kann als Bohrung ausgeführt sein. Vorzugsweise weist der zweite Durchgang einen runden, insbesondere kreisförmigen, oder einen eckigen Querschnitt auf. Eine medizinische Fluidsteuervorrichtung mit einem derartigen Steuerkörper bzw. mit einem derartigen ersten Fluidkanal ist besonders einfach herstellbar. Insbesondere ist eine uneinheitliche Querschnittsgestaltung möglich, so dass sich der Querschnitt über eine Erstreckung des zweiten Durchgangs ändert.

In weiterer Ausgestaltung der Erfindung umfasst der Steuerkörper eine zweite Nut, die an der Außenumfangsfläche des Steuerkörpers abschnittsweise ausgenommen ist und durch welche der zweite Fluidkanal gebildet ist. Dies geht einher mit einer besonders geringen mechanischen Schwächung des Steuerkörpers.

In weiterer Ausgestaltung der Erfindung umfasst das Gehäuse eine zweite Nut, die an einer Innenumfangsfläche der Aufnahmeaussparung abschnittsweise ausgenommen ist, wobei der zweite Fluidkanal durch die zweite Nut gebildet ist. Somit ergibt sich für das Material des Steuerkörpers aus der Führung des zweiten Fluidkanals keinerlei mechanische Schwächung, was sich besonders als vorteilhaft erweist, wenn der Steuerkörper als, insbesondere dünnwandiger, Hohlkörper realisiert wird.

In weiterer Ausgestaltung der Erfindung umfasst der Steuerkörper eine zweite Nut, die an seiner Außenumfangsfläche abschnittsweise ausgenommen ist, wobei das Gehäuse eine zweite Gehäusenut umfasst, die an einer Innenumfangsfläche der Aufnahmeaussparung abschnittsweise ausgenommen ist, wobei der zweite Fluidkanal durch die zweite Nut und durch die zweite Gehäusenut gebildet ist. Die zweite Gehäusenut kann die zweite Nut zur gemeinsamen Führung des zweiten Fluidkanals, insbesondere in der zweiten Schaltstellung, komplettieren. Eine derart ausgestaltete medizinische Fluidsteuervorrichtung baut besonders kompakt. Zudem können auf diese Weise geometrisch besonders komplexe Verläufe des zweiten Fluidkanals realisiert werden.

In weiterer Ausgestaltung der Erfindung ist in dem ersten Fluidkanal ein Widerstandselement sowie - alternativ oder zusätzlich - eine Engstelle, die insbesondere durch eine Blende ausgebildet sein kann, angeordnet. Somit lässt sich ein besonders großer erster Strömungswiderstand des ersten Fluidkanals erreichen. Dies wiederum erlaubt es, in der ersten Schaltstellung des Steuerkörpers besonders geringe und zugleich konstante Volumenströme an durch die Steuervorrichtung geführtem Fluid zu realisieren. Ein derartiger besonders geringer und zugleich konstanter Volumenstrom ist insbesondere wünschenswert für die Applikation bestimmter Medikamente oder zur Offenhaltung der medizinischen Fluidsteuervorrichtung während einer Applikationspause. Vorgenannte Widerstandselemente können in verschiedener Spezifikation baukastenartig vorgehalten werden und je nach Therapieansatz in den ersten Fluidkanal eingebracht werden, um den ersten Strömungswiderstand anforderungsgerecht anzupassen. Zwecks Anpassung können mehrere Widerstandselemente nach Art einer Reihenschaltung miteinander kombiniert werden.

In weiterer Ausgestaltung der Erfindung ist in dem zweiten Fluidkanal ein Widerstandselement sowie - alternativ oder zusätzlich - eine Engstelle, die insbesondere durch eine Blende ausgebildet sein kann, angeordnet. Folglich lässt sich ein besonders großer zweiter Strömungswiderstand des zweiten Fluidkanals erreichen. Die Widerstandselemente können in verschiedener Spezifikation baukastenartig vorgehalten werden. Zur Vermeidung von Wiederholungen wird auf die vorhergehende Ausgestaltung verwiesen. Das dort Offenbarte gilt mutatis mutandis auch vorliegend.

In weiterer Ausgestaltung der Erfindung umfasst das Widerstandselement einen Grundkörper, der insbesondere eine zylindrische Formgebung aufweist. Der Grundkörper umfasst ein fluiddurchlässiges poröses Material, insbesondere ein Sintermaterial, oder besteht aus einem derartigen Material. Alternativ oder zusätzlich weist der Grundkörper wenigstens eine, vorzugsweise mehrere, beidenends geöffnete fluiddurchlässige Kapillare auf. Besonders bevorzugt weisen alle vorhandenen Kapillaren zusammen eine fluiddurchlässige Gesamtquerschnittsfläche von 7*10⁻⁵ mm² bis 7.04 mm², höchst vorzugsweise von 7*10⁻⁵ mm² bis 3.14 mm² auf. Alternativ oder zusätzlich weist der Grundkörper eine Außenfläche auf, zwischen welcher und einer den jeweiligen Fluidkanal begrenzenden Kanalwand wenigstens ein fluiddurchlässiger Zwischenraum ausgebildet ist, wobei vorzugsweise an der Außenfläche eine rückspringende Rille zur Ausbildung des fluiddurchlässigen Zwischenraums vorhanden ist. Besonders bevorzugt weisen alle vorhandenen Rillen gemeinsam eine fluiddurchlässige Gesamtquerschnittsfläche von 7*10⁻⁵ mm² bis 7.04 mm², höchst vorzugsweise von 7*10⁻⁵ mm² bis 3.14mm², auf. Mittels eines derartigen Widerstandselements lässt sich der Strömungswiderstand des jeweiligen Fluidkanals, in welchem das Widerstandselement angeordnet ist, besonders präzise beeinflussen oder festlegen.

Zweckmäßig ist die Engstelle oder/und das Widerstandselement integral an dem jeweiligen den ersten bzw. zweiten Fluidkanal begrenzenden Bauteil ausgebildet. Besonders bevorzugt ist die Engstelle oder/und das Widerstandselement mittels eines Laserbearbeitungsverfahrens erzeugt. Dies vermeidet vorteilhaft die Gefahr einer fehlerhaften Montage, insbesondere die versehentliche Auswahl eines für den gewünschten Therapie ungeeigneten Widerstandselements.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind. Dabei beziehen sich gleiche Bezugszeichen auf gleiche oder ähnliche oder funktional gleiche Bauteile.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.
- Fig. 1: zeigt in einem schematischen Mittellängsschnitt eine Ausführungsform einer erfindungsgemäßen medizinischen Fluidsteuervorrichtung in einer ersten Schaltstellung,
- Fig. 2: ebenfalls in schematischem Mittellängsschnitt die medizinische Fluidsteuervorrichtung nach Fig. 1 in einer zweiten Schaltstellung,
- Fig. 3: in einem schematischen Querschnitt die medizinische Fluidsteuervorrichtung nach Fig. 1 und 2 in einer dritten Schaltstellung,
- Fig. 4: in einer schematischen Perspektivdarstellung die medizinische Fluidsteuervorrichtung in der ersten Schaltstellung,
- Fig. 5: in einer schematischen Perspektivdarstellung die medizinische Fluidsteuervorrichtung in der zweiten Schaltstellung,
- Fig. 6: in einer schematischen Perspektivdarstellung die medizinische Fluidsteuervorrichtung in der dritten Schaltstellung,
- Fig. 7: in einer schematischen Perspektivdarstellung einen Steuerkörper der medizinischen Fluidsteuervorrichtung,
- Fig. 8: in einem schematischen Mittellängsschnitt den Steuerkörper der Fig. 7,
- Fig. 9: in einer schematischen Seitenansicht den Steuerkörper einer weiteren Ausführungsform einer erfindungsgemäßen medizinischen Fluidsteuervorrichtung,
- Fig 10: in einer weiteren, gegenüber der Darstellung der Fig. 9 gedrehten schematischen Seitenansicht den Steuerkörper nach Fig. 9,
- Fig. 11: in schematischer Perspektivdarstellung ein zur Veranschaulichung teils ausgebrochenes Gehäuse einer weiteren Ausführungsform einer erfindungsgemäßen medizinischen Fluidsteuervorrichtung,
- Fig. 12: in einem schematischen Mittellängsschnitt eine weitere Ausführungsform der medizinischen Fluidsteuervorrichtung in der zweiten Schaltstellung,
- Fig. 13: in einem teilweise abgeschnittenen Querschnitt die medizinische Fluidsteuervorrichtung nach Fig. 12,
- Fig. 14: in einem schematischen Querschnitt den Steuerkörper der medizinischen Fluidsteuervorrichtung nach den Fig. 1 bis 3,
- Fig. 15: in einem schematischen Querschnitt das Gehäuse der medizinischen Fluidsteuervorrichtung nach den Fig. 1 bis 3,
- Fig. 16: den Steuerkörper einer weiteren Ausführungsform einer erfindungsgemäßen medizinischen Fluidsteuervorrichtung in einem Detail eines schematischen Mittellängsschnitts, und
- Fig. 17 bis: 21 verschiedene Widerstandselemente für weitere Ausführungsformen erfindungsgemäßer medizinischer Fluidsteuervorrichtungen.

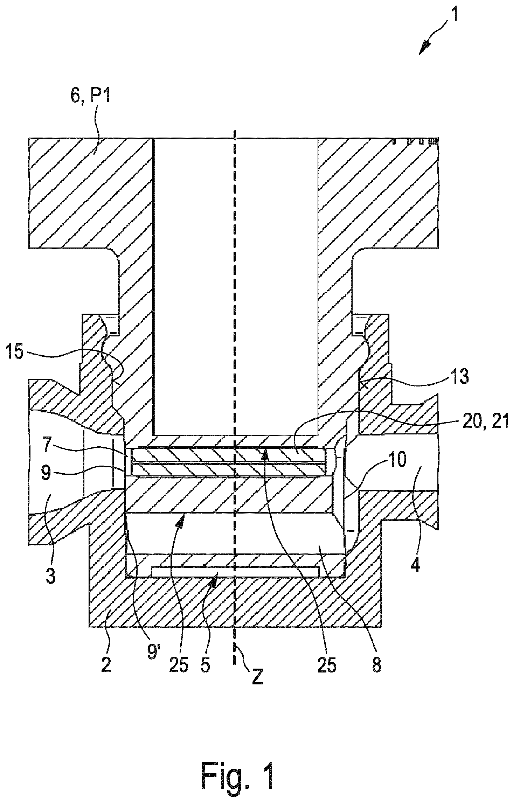

Eine medizinische Fluidsteuervorrichtung 1 gemäß den Fig. 1, 2 und 3 ist zur Fluidsteuerung in einem medizinischen Fluidleitungssystem vorgesehen. Bei einem solchen Fluidleitungssystem kann es sich beispielsweise um ein Schwerkraftinfusionssystem oder ein Druckinfusionssystem mit einer Pumpe handeln. Dabei erlaubt es die medizinische Fluidsteuervorrichtung 1, verschiedene vordefinierte und auf einen Anwendungszweck abgestimmte fixe Flussraten eines durch das Schwerkraftinfusionssystem und durch die medizinische Fluidsteuervorrichtung 1 geführten Fluidstroms zu realisieren. Mit anderen Worten ermöglicht es die medizinische Fluidsteuervorrichtung 1 bei gegebener über die Fluidsteuervorrichtung 1 abfallender Druckdifferenz vorbestimmte Volumenströme stufenweise einzustellen.

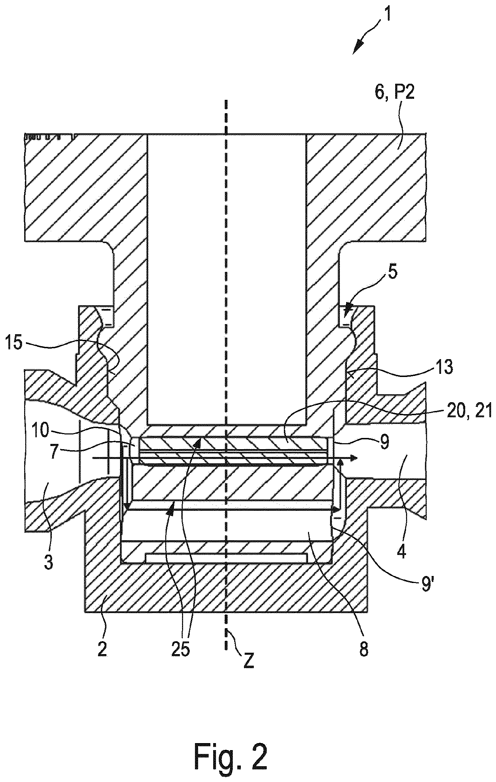

Die medizinische Fluidsteuervorrichtung 1 weist ein Gehäuse 2 mit einem Einlass 3 und einem Auslass 4 auf. Bei der gezeigten Ausführungsform weist das Gehäuse zudem eine Aufnahmeaussparung 5 auf. Die medizinische Fluidsteuervorrichtung 1 umfasst ferner einen Steuerkörper 6. Der Steuerkörper 6 ist wenigstens zwischen einer ersten Schaltstellung P1 - gezeigt in Fig. 1 - und einer zweiten Schaltstellung P2 - gezeigt in Fig. 2 - relativ zu dem Gehäuse 2 um eine Drehachse Z drehbeweglich. Vorliegend ist der Steuerkörper 6 drehbeweglich in der Aufnahmeaussparung 5 aufgenommen. Der Steuerkörper 6 kann als Drehschieber ausgebildet sein. Die medizinische Fluidsteuervorrichtung 1 weist darüber hinaus wenigstens einen ersten Fluidkanal 7 mit einem ersten Strömungswiderstand und einen zweiten Fluidkanal 8 mit einem zweiten Strömungswiderstand auf. Dabei ist der erste Fluidkanal 7 an dem Steuerkörper 6 sowie - alternativ oder zusätzlich - an dem Gehäuse 2 ausgebildet. Bei der medizinischen Fluidsteuervorrichtung 1 der Fig. 1 bis 3 ist der erste Fluidkanal 7 vollständig an dem Steuerkörper 6 ausgebildet. Der zweite Fluidkanal 8 ist an dem Steuerkörper 6 sowie - alternativ oder zusätzlich - an dem Gehäuse 2 ausgebildet. Bei der medizinischen Fluidsteuervorrichtung 1 der Fig. 1 bis 3 ist der zweite Fluidkanal 8 vollständig an dem Steuerkörper 6 ausgebildet. Im Betrieb der Fluidsteuervorrichtung 1 wird die Fluidsteuervorrichtung 1 von ihrem Einlass 3 zu ihrem Auslass 4 hin von einem Fluid durchströmt. Es versteht sich, dass auch eine Durchströmung in umgekehrter Richtung möglich ist, so dass dann der Einlass 3 als Auslass und der Auslass 4 als Einlass der Fluidsteuervorrichtung 1 fungiert.

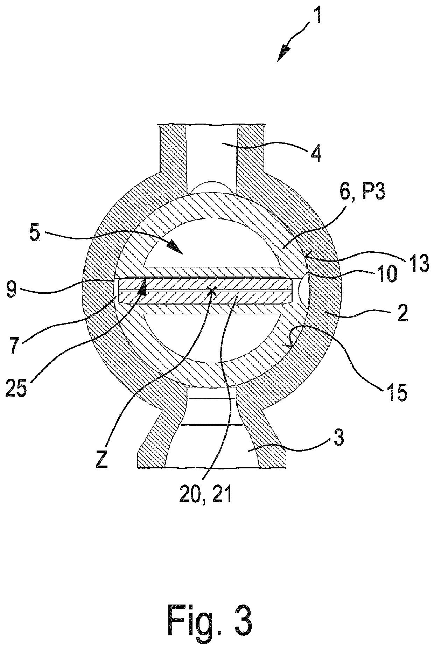

Wie die Fig. 1 erkennen lässt, sind in der ersten Schaltstellung P1 der Einlass 3 und der Auslass 4 mittels des ersten Fluidkanals 7 fluidleitend miteinander verbunden. Demgegenüber ist der Fig. 2 zu entnehmen, dass in der zweiten Schaltstellung P2 der Einlass 3 und der Auslass 4 mittels des zweiten Fluidkanals 8 fluidleitend miteinander verbunden sind. Der Steuerkörper 6 der medizinischen Fluidsteuervorrichtung 1 der Fig. 1 bis 3 lässt sich zusätzlich zu den in den Fig. 1 und 2 gezeigten Schaltstellungen P1, P2 in eine dritte Schaltstellung P3 relativ zum Gehäuse 2 drehverstellen. Dies ist in der Fig. 3 dargestellt. In der dritten Schaltstellung P3 sind der Einlass 3 und der Auslass 4 mittels des Steuerkörpers 6 fluiddicht voneinander getrennt. Die dritte Schaltstellung P3 befindet sich vorliegend zwischen der ersten und der zweiten Schaltstellung P1, P2, d. h. ein Drehverstellen des Steuerkörpers 6 aus der ersten Schaltstellung P1 in die zweite Schaltstellung P2 erfolgt stets über die dritte Schaltstellung P3. In der Ausführungsform der Fig. 1 bis 3 ist der Steuerkörper 6 in der zweiten Schaltstellung P2 relativ zur ersten Schaltstellung P1 um 180° bezogen auf die Drehachse Z drehverstellt. In der dritten Schaltstellung P3 ist der Steuerkörper 6 relativ zur ersten und zur zweiten Schaltstellung P1, P2 um 90° oder um 270° drehverstellt. Es versteht sich, dass alternativ hierzu bei weiteren und in den Figuren nicht gezeigten Ausführungsformen der Fluidsteuervorrichtung 1 auch andere den einzelnen Schaltstellungen P1, P2, P3 zugehörige Stellwinkel möglich sind.

Aus den Fig. 1 und 2 ergibt es sich außerdem, dass der erste Fluidkanal 7 und der zweite Fluidkanal 8 zwischen jeweils einer ersten Mündungsöffnung 9, 9' und einer gemeinsamen zweiten Mündungsöffnung 10 erstreckt sind. Die erste Mündungsöffnung 9 ist dem ersten Fluidkanal 7 zugeordnet. Die erste Mündungsöffnung 9` ist dem zweiten Fluidkanal 8 zugeordnet. Die gemeinsame zweite Mündungsöffnung 10 kann als Öffnung eines Sammlers bzw. Verteilers aufgefasst werden, mit welchem die beiden Fluidkanäle 7, 8 fluidleitend kommunizieren. Dabei mündet in der ersten Schaltstellung P1 der Einlass 3 in die erste Mündungsöffnung 9 des ersten Fluidkanals 7 und von dort über den ersten Fluidkanal 7 und die gemeinsame zweite Mündungsöffnung 10 in den Auslass 4. Demgegenüber mündet in der zweiten Schaltstellung P2 der Einlass 3 in die gemeinsame zweite Mündungsöffnung 10 und von dort über die beiden Fluidkanäle 7, 8 und die jeweilige erste Mündungsöffnung 9, 9` in den Auslass 4.

Gemäß Fig. 2 sind in der zweiten Schaltstellung P2 der Einlass 3 und der Auslass 4 sowohl mittels des ersten Fluidkanals 7 als auch mittels des zweiten Fluidkanals 8 fluidleitend miteinander verbunden. Dabei sind der erste Fluidkanal 7 und der zweite Fluidkanal 8 in der zweiten Schaltstellung P2 fluidleitend parallelgeschaltet. Während in der zweiten Schaltstellung P2 die ersten Mündungsöffnungen 9, 9' beider Fluidkanäle 7, 8 zum Auslass 4 hin geöffnet sind, ist in der ersten Schaltstellung P1 die erste Mündungsöffnung 9` des zweiten Fluidkanals 8 fluiddicht verschlossen, siehe Fig. 1 in Zusammenschau mit Fig. 2. Dabei ist ein zwischen dem Ein- und dem Auslass 3, 4 vorhandener und von einem Fluid durchströmbarer Gesamt-Strömungswiderstand in der ersten Schaltstellung P1 größer als in der zweiten Schaltstellung P2. Somit stellt sich, wenn die medizinische Fluidsteuervorrichtung im Betrieb von ihrem Ein- zu ihrem Auslass 3, 4 hin von einem Fluid durchströmt wird, in der ersten Schaltstellung P1 ein geringerer durch die Fluidsteuervorrichtung 1 fließender Fluidvolumenstrom ein als in der zweiten Schaltstellung P2. Beispielsweise kann die erste Schaltstellung P1 während einer Medikamentengabe genutzt werden. Demgegenüber ist die zweite Schaltstellung P2 für einen Spülvorgang zum Reinigen des medizinischen Fluidleitungssystems bzw. der medizinischen Fluidsteuervorrichtung nutzbar.

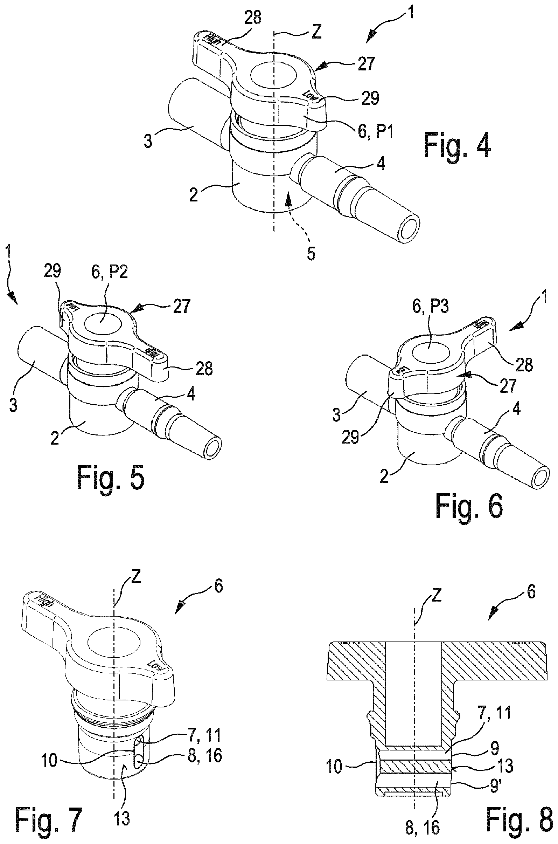

Vorliegend umfasst der Steuerkörper 6 zudem einen Handgriff 27, mittels welchem der Steuerkörper 6 zwischen seinen Schaltstellungen P1, P2, P3 manuell drehverstellbar ist. Um einem Bediener der medizinischen Fluidsteuervorrichtung 1 anzuzeigen, in welcher Schaltstellung P1, P2, P3 sich der Steuerkörper 6 momentan befindet, verfügt der Handgriff 27 über einen langen Flügel 28 und über einen kurzen Flügel 29. Befindet sich der lange Flügel 28 über dem Einlass 3, so zeigt dies im vorliegenden Fall dem Benutzer an, dass sich der Steuerkörper 6 in seiner ersten Schaltstellung P1 befindet, vgl. Fig. 4. Steht der kurze Flügel 29 über dem Einlass 3 - wie dies in der Fig. 5 gezeigt ist -, so signalisiert dies dem Benutzer, dass sich der Steuerkörper 6 in seiner zweiten Position P2 befindet. Sind wie in Fig. 6 weder der kurze Flügel 28 noch der lange Flügel 29 des Handgriffs 27 entlang dem Einlass 3 bzw. dem Auslass 4 angeordnet, so zeigt dies dem Benutzer an, dass sich der Steuerkörper 6 in seiner dritten Position P3 befindet. Es versteht sich, dass eine derartige Anzeige der momentanen Schaltstellung P1, P2, P3 auch mit anderen Markierungen am Steuerkörper 6 und am Gehäuse 2 realisiert sein kann. Alternativ oder zusätzlich kann dabei das Gehäuse 2 sowie gegebenenfalls der Steuerkörper 6 selbst aus einem transparenten Material hergestellt sein, sodass ein Innenleben der Fluidsteuervorrichtung 1 von außen einsehbar ist.

Die Fig. 7 bis 10 illustrieren, wie die beiden Fluidkanäle 7, 8 bei verschiedenen weiteren Ausführungsformen der medizinischen Fluidsteuervorrichtung 1 gestaltet sind.

Gemäß den Fig. 7 und 8 umfasst der Steuerkörper 6 einen ersten Durchgang 11, durch welchen der erste Fluidkanal 7 gebildet ist, sowie einen zweiten Durchgang 16, durch welchen der zweite Fluidkanal 8 gebildet ist. Dabei weisen die Durchgänge 11, 16 über ihre Erstreckung einen konstanten kreisförmigen Querschnitt auf. Mit anderen Worten sind die Durchgänge 11, 16 als Bohrungen ausgeführt. Alternative Querschnittsformen sind denkbar. Die beiden Durchgänge 11, 16 sind bei der Ausführungsform der Fig. 7 und 8 je gerade längserstreckt. Es versteht sich, dass alternativ auch unterschiedlich gestaltete, beispielsweise kurvenförmige oder abgewinkelte, Erstreckungen der Durchgänge möglich sind. In den Fig. 7 und 8 verlaufen die beiden Durchgänge 11, 16 parallel zueinander. Alternativ können die Durchgänge 11, 16 in weiteren und in den Figuren nicht gezeigten Ausführungsformen wenigstens abschnittsweise winkelig gegeneinander angestellt sein.

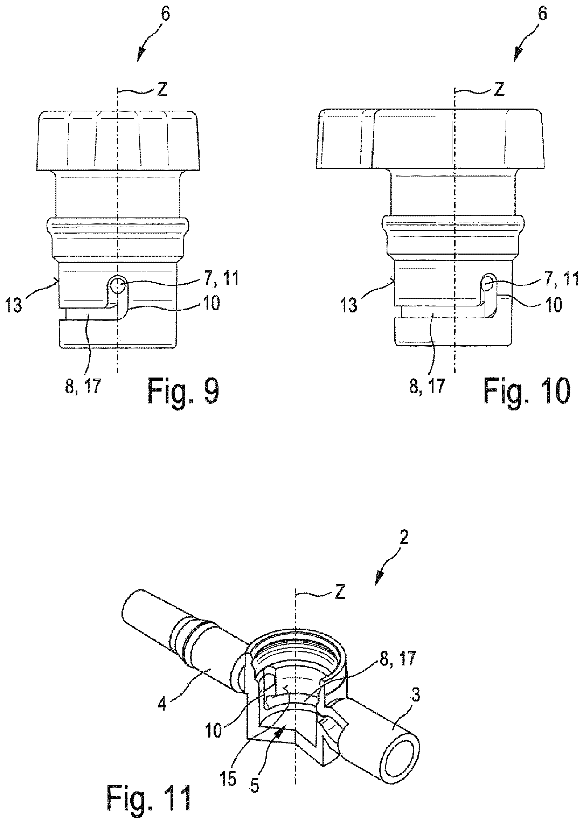

Bei einer anderen Ausführungsform der medizinischen Fluidsteuervorrichtung 1, deren Steuerkörper 6 in den Fig. 9 und 10 gezeigt ist, umfasst der Steuerkörper 6 einen ersten Durchgang 11, durch welchen der erste Fluidkanal 7 gebildet ist. Der erste Durchgang 11 ist bei der gezeigten Ausführungsform als Bohrung ausgebildet. Bei einer in den Figuren nicht gezeigten Ausführungsform umfasst der Steuerkörper 6 - alternativ oder zusätzlich zum ersten Durchgang 11 - eine erste Nut, die an einer Außenumfangsfläche 13 des Steuerkörpers 6 abschnittsweise ausgenommen ist und durch welche der erste Fluidkanal 7 gebildet ist. Bei einer weiteren in den Figuren nicht gezeigten Ausführungsform umfasst das Gehäuse 2 - alternativ oder zusätzlich zum am Steuerkörper 6 vorhandenen ersten Durchgang 11 und alternativ oder zusätzlich zur am Steuerkörper 6 vorhandenen ersten Nut - eine erste Nut, die an einer Innenumfangsfläche 15 ausgenommen ist, wobei der erste Fluidkanal 7 durch die erste Nut des Gehäuses 2 gebildet ist. Bei einer weiteren in den Figuren nicht gezeigten Ausführungsform umfasst der Steuerkörper 6 - alternativ oder zusätzlich zum ersten Durchgang 11 - eine erste Nut, die an seiner Außenumfangsfläche 13 abschnittsweise ausgenommen ist, wobei das Gehäuse 2 eine erste Gehäusenut umfasst, die an der Innenumfangsfläche 15 der Aufnahmeaussparung 5 abschnittsweise ausgenommen ist, wobei der erste Fluidkanal 7 durch die erste Nut und durch die erste Gehäusenut gebildet ist.

Wie bereits erläutert lassen die Fig. 7 und 8 erkennen, dass der Steuerkörper 6 einen zweiten Durchgang 16 umfasst, durch welchen der zweite Fluidkanal 8 gebildet ist. Im Unterschied hierzu umfasst der Steuerkörper 6 bei der Ausführungsform der Fig. 9 und 10 eine zweite Nut 17, die an der Außenumfangsfläche 13 des Steuerkörpers 6 abschnittsweise ausgenommen ist und durch welche der zweite Fluidkanal 8 gebildet ist. Es versteht sich, dass in einer weiteren und in den Figuren nicht gezeigten Ausführungsform auch eine Kombination aus zweiter Nut 17 und zweitem Durchgang 16 zur Führung des zweiten Fluidkanals 8 realisiert sein kann.

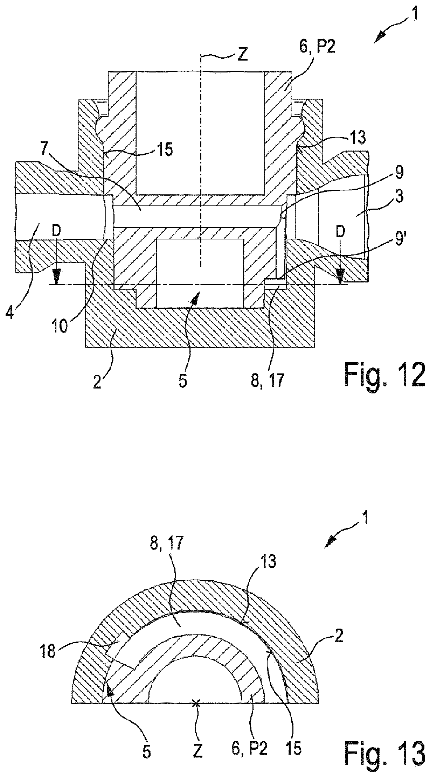

Gemäß der weiteren Ausführungsform der Fig. 11 ist das Gehäuse 2 mit einer (zweiten) Nut 17 ausgeführt, die an einer Innenumfangsfläche 15 der Aufnahmeaussparung 5 abschnittsweise ausgenommen ist und durch welche der zweite Fluidkanal 8 gebildet ist. Demgegenüber umfasst der Steuerkörper 6 bei der Ausführungsform der Fig. 12 und 13 die zweite Nut 17, die an der Außenumfangsfläche 13 des Steuerkörpers 6 abschnittsweise ausgenommen ist. Dabei umfasst das Gehäuse 2 bei der Ausführungsform der Fig. 12 und 13 eine zweite Gehäusenut 18, die an der Innenumfangsfläche 15 der Aufnahmeaussparung 5 abschnittsweise ausgenommen ist. Gemäß den Fig. 12 und 13 ist der zweite Fluidkanal 8 sowohl durch die zweite Nut 17 als auch durch die zweite Gehäusenut 18 gebildet.

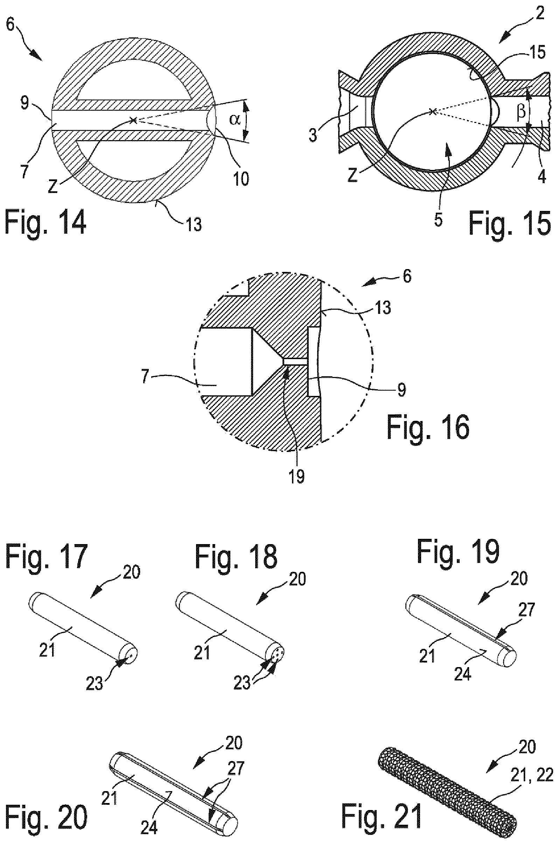

Vorliegend ist in dem ersten Fluidkanal 7 ein Widerstandselement 20 angeordnet. Alternativ oder zusätzlich kann eine Engstelle 19 vorhanden sein, wie dies exemplarisch bei der Ausführungsform der Fig. 16 gezeigt ist. Auch in dem zweiten Fluidkanal 8 kann ein Widerstandselement 20 sowie - alternativ oder zusätzlich - eine Engstelle 19 angeordnet sein, was in den Figuren jedoch nicht gezeigt ist. Die Engstelle 19 ist gemäß Fig. 16 integral an dem Steuerkörper 6 ausgeformt. Alternativ kann zur Ausbildung der Engstelle 19 auch eine Blende mit der Engstelle 19 am Steuerkörper 6 vorgesehen sein. Die Engstelle 19 kann integral an dem den jeweiligen Fluidkanal 7, 8 begrenzenden Bauteil ausgeformt sein. Die Engstelle 19 kann mittels eines Laserbearbeitungsverfahrens erzeugt sein.

Die Fig. 17 bis 21 zeigen verschiedene Ausführungsformen von Widerstandselementen 20. Diese Widerstandselemente 20 können in dem ersten sowie - alternativ oder zusätzlich - zweiten Fluidkanal 7, 8 weiterer Ausführungsformen der medizinischen Fluidsteuervorrichtung 1 angeordnet werden. Das Widerstandselement 20 der Fig. 17 entspricht dabei jenem, welches bei der medizinischen Fluidsteuervorrichtung der Fig. 1 bis 3 in dem ersten Fluidkanal 7 angeordnet ist. Demnach umfasst das Widerstandselement 20 einen Grundkörper 21. Bei den gezeigten Ausführungsformen weist der Grundkörper 21 eine kreiszylindrische Formgebung auf. Der Grundkörper 21 kann stiftförmig ausgebildet sein. Bei einer Ausführungsform umfasst der Grundkörper 21 ein poröses Material 22 oder besteht aus einem solchen fluiddurchlässigen porösen Material 22, gezeigt in Fig. 21. Ein derartiges fluiddurchlässiges poröses Material 22 kann ein Sintermaterial sein. Bei der Ausführungsform der Fig. 17 weist der Grundkörper 21 eine beidseits geöffnete fluiddurchlässige Kapillare 23 auf. Gemäß der Ausführungsform der Fig. 18 sind mehrere - gezeigt sind fünf - solcher Kapillaren 23 vorhanden. Die wenigstens eine oder alle vorhandenen Kapillaren 23 zusammen weist/weisen eine fluiddurchlässige Gesamtquerschnittsfläche von 7*10⁻⁵ mm² bis 7.04 mm². Die Gesamtquerschnittsfläche kann 7*10⁻⁵ mm² bis 3.14mm² betragen. Bei der Ausführungsform der Fig. 19 weist der Grundkörper 21 eine Außenfläche 24 auf, zwischen welcher und einer den jeweiligen Fluidkanal 7, 8 begrenzenden Kanalwand 25 (erkennbar in Fig. 1 bis 3) ein fluiddurchlässiger Zwischenraum ausgebildet ist. Gemäß Fig. 19 ist zur Ausbildung des fluiddurchlässigen Zwischenraums an der Außenfläche 24 des Grundkörpers 21 eine rückspringende Rille 27 vorhanden. Die Rille 27 erstreckt sich in Fig. 19 über eine gesamte Länge des Widerstandselements 20. Bei der Ausführungsform gemäß Fig. 20 sind mehrere derartige Rillen 27 vorgesehen. Wenigstens eine solche Rille 27 oder alle Rillen 27 gemeinsam kann/können eine fluiddurchlässige Gesamtquerschnittsfläche von 7*10⁻⁵ mm² bis 7.04 mm² aufweisen. Beispielsweise kann die Gesamtdurchschnittsfläche 7*10⁻⁵ mm² bis 3.14 mm² betragen.

Es versteht sich, dass das fluiddurchlässige poröse Material 22 sowie - alternativ oder zusätzlich - die wenigstens eine beidenends geöffnete fluiddurchlässige Kapillare 23 sowie - alternativ oder zusätzlich - der fluiddurchlässige Zwischenraum zwischen der Außenfläche 24 des Grundkörpers 21 des Widerstandselements 20 und der Kanalwand 25 an ein- und demselben Widerstandselement 20 realisiert sein kann. Mit anderen Worten: Bei weiteren in den Figuren nicht gezeigten Ausführungsformen können die vorstehend beschriebenen und in den Fig. 17 bis 21 gezeigten Merkmale der Widerstandselemente 20 in Alleinstellung oder in Kombination realisiert sein. Ebenso ist es denkbar, mehrere Widerstandselemente 20 gleicher oder verschiedener Spezifikation nach der Art einer Reihenschaltung hintereinander in dem jeweiligen Fluidkanal 7, 8 anzuordnen. Das Widerstandselement 20 kann in weiteren Ausführungsformen integral an dem den jeweiligen Fluidkanal 7, 8 begrenzenden Bauteil ausgeformt sein. Das Widerstandselement 20 kann mittels eines Laserbearbeitungsverfahrens erzeugt sein.

Die Fig. 14 lässt erkennen, dass sich die gemeinsame zweite Öffnung 10 der beiden Fluidkanäle 7, 8 entlang der Drehachse Z betrachtet innerhalb eines Winkels α, dessen Scheitel auf der Drehachse Z des Steuerkörpers 6 angeordnet ist, erstreckt. Dabei ist der Winkel α kleiner 90°. Gemäß Fig. 15 erstreckt sich eine Öffnung des Auslasses 4 entlang der Drehachse Z des Steuerkörpers 6 betrachtet innerhalb eines Winkels β, dessen Scheitel auf der Drehachse Z angeordnet ist. Der Winkel β beträgt weniger als 90°. Die Winkel α und β können gleich groß sein.

Bei den Ausführungsformen der Fig. 1 bis 3, 7 bis 12 ist erkennbar, dass die gemeinsame Mündungsöffnung 10 der beiden Fluidkanäle 7, 8 durch eine erste Ausnehmung gebildet ist, die von der Außenumfangsfläche 13 des Steuerkörpers 6 rückspringt. Der Auslass 4 umfasst eine zweite Ausnehmung, die von der Innenumfangsfläche 15 des Gehäuses 2 rückspringt. Die beiden Ausnehmungen sind so aufeinander abgestimmt, dass sie in der ersten Schaltstellung P1 unmittelbar fluidleitend miteinander verbunden sind. Die beiden Ausnehmungen sind ferner derart aufeinander abgestimmt, dass sie in der zweiten Schaltstellung P2 mittels des ersten Fluidkanals 7 - sowie alternativ oder zusätzlich - des zweiten Fluidkanals 8 fluidleitend miteinander verbunden sind. Die beiden Ausnehmungen sind außerdem so aufeinander abgestimmt, dass sie in der dritten Schaltstellung P3 fluiddicht voneinander getrennt sind. In der zweiten Schaltstellung P2 können die beiden Ausnehmungen zusammen mit dem zweiten Fluidkanal 8 einen Bypass ausbilden, der fluidleitend parallel zu dem ersten Fluidkanal 7 geschaltet ist. Ein Teil des im Betrieb der Fluidsteuervorrichtung 1 vom Ein- zum Auslass 3, 4 geleiteten Fluids kann also an dem ersten Fluidkanal 7 vorbei über den Bypass geführt werden. Eine an der Innenumfangsfläche 15 des Gehäuses 2 angeordnete Öffnung des Einlasses 3 ist kleiner als eine die zweite Ausnehmung an der Innenumfangsfläche 15 einfassende Öffnung des Auslasses 4.

## Patentansprüche

1. Medizinische Fluidsteuervorrichtung (1) für ein medizinisches Fluidleitungssystem, aufweisend
ein Gehäuse (2) mit einem Einlass (3), mit einem Auslass (4) und mit einer Aufnahmeaussparung (5),
einen Steuerkörper (6), welcher wenigstens zwischen einer ersten Schaltstellung (P1) und einer zweiten Schaltstellung (P2) relativ zu dem Gehäuse (2) um eine Drehachse (Z) drehbeweglich in der Aufnahmeaussparung (5) aufgenommen ist,
wenigstens einen ersten Fluidkanal (7) mit einem ersten Strömungswiderstand und einen zweiten Fluidkanal (8) mit einem zweiten Strömungswiderstand, wobei der erste Fluidkanal (7) und der zweite Fluidkanal (8) jeweils an dem Steuerkörper (6) und/oder an dem Gehäuse (2) ausgebildet sind,
wobei in der ersten Schaltstellung (P1) der Einlass (3) und der Auslass (4) mittels des ersten Fluidkanals (7) fluidleitend miteinander verbunden sind,
und wobei in der zweiten Schaltstellung (P2) der Einlass (3) und der Auslass (4) mittels des ersten Fluidkanals (7) und des zweiten Fluidkanals (8) fluidleitend miteinander verbunden sind, wobei der erste Fluidkanal (7) und der zweite Fluidkanal (8) fluidleitend parallelgeschaltet sind.

2. Medizinische Fluidsteuervorrichtung (1) nach Anspruch 1, **dadurch gekennzeichnet, dass** eine dritte Schaltstellung (P3) vorhanden ist, in welcher der Einlass (3) und der Auslass (4) mittels des Steuerkörpers (6) fluiddicht voneinander getrennt sind.

3. Medizinische Fluidsteuervorrichtung (1) nach Anspruch 1 oder 2, **dadurch gekennzeichnet, dass** der erste Fluidkanal (7) und der zweiten Fluidkanal (8) zwischen jeweils einer ersten Mündungsöffnung (9, 9`) und einer gemeinsamen zweiten Mündungsöffnung (10) erstreckt sind, wobei in der ersten Schaltstellung (P1) der Einlass (3) in die erste Mündungsöffnung (9) des ersten Fluidkanals (7) und von dort über den ersten Fluidkanal (7) und die gemeinsame zweite Mündungsöffnung (10) in den Auslass (4) mündet, und wobei in der zweiten Schaltstellung (P2) der Einlass (3) in die gemeinsame zweite Mündungsöffnung (10) und von dort über die beiden Fluidkanäle (7, 8) und die jeweilige erste Mündungsöffnung (9, 9`) in den Auslass (4) mündet.

4. Medizinische Fluidsteuervorrichtung (1) nach Anspruch 3, **dadurch gekennzeichnet, dass** in der ersten Schaltstellung (P1) die erste Mündungsöffnung (9`) des zweiten Fluidkanals (8) fluiddicht verschlossen ist.

5. Medizinische Fluidsteuervorrichtung (1) nach einem der vorhergehenden Ansprüche, **dadurch gekennzeichnet, dass** ein zwischen dem Ein- und dem Auslass (3, 4) vorhandener und durchströmbarer Gesamt-Strömungswiderstand in der ersten Schaltstellung (P1) größer ist als in der zweiten Schaltstellung (P2).

6. Medizinische Fluidsteuervorrichtung (1) nach einem der vorhergehenden Ansprüche, **dadurch gekennzeichnet, dass** der Steuerkörper (6) einen ersten Durchgang (11) umfasst, durch welchen der erste Fluidkanal (7) gebildet ist; oder dass der Steuerkörper (6) eine erste Nut umfasst, die an einer Außenumfangsfläche (13) des Steuerkörpers (6) abschnittsweise ausgenommen ist und durch welche der erste Fluidkanal (7) gebildet ist; oder dass das Gehäuse (2) eine erste Nut umfasst, die an einer Innenumfangsfläche (15) der Aufnahmeaussparung (5) abschnittsweise ausgenommen ist, wobei der erste Fluidkanal (7) durch die erste Nut gebildet ist; oder dass der Steuerkörper (6) eine erste Nut umfasst, die an seiner Außenumfangsfläche (13) abschnittsweise ausgenommen ist, wobei das Gehäuse (2) eine erste Gehäusenut umfasst, die an der Innenumfangsfläche (15) der Aufnahmeaussparung (5) abschnittsweise ausgenommen ist, wobei der erste Fluidkanal (7) durch die erste Nut und durch die erste Gehäusenut gebildet ist.

7. Medizinische Fluidsteuervorrichtung (1) nach einem der vorhergehenden Ansprüche, **dadurch gekennzeichnet, dass** der Steuerkörper (6) einen zweiten Durchgang (16) umfasst, durch welchen der zweite Fluidkanal (8) gebildet ist; oder dass der Steuerkörper (6) eine zweite Nut (17) umfasst, die an der Außenumfangsfläche (13) des Steuerkörpers (6) abschnittsweise ausgenommen ist und durch welche der zweite Fluidkanal (8) gebildet ist; oder dass das Gehäuse (2) eine zweite Nut (17) umfasst, die an einer Innenumfangsfläche (15) der Aufnahmeaussparung (5) abschnittsweise ausgenommen ist, wobei der zweite Fluidkanal (8) durch die zweite Nut (17) gebildet ist; oder dass der Steuerkörper (6) eine zweite Nut (17) umfasst, die an seiner Außenumfangsfläche (13) abschnittsweise ausgenommen ist, wobei das Gehäuse (2) eine zweite Gehäusenut (18) umfasst, die an einer Innenumfangsfläche (15) der Aufnahmeaussparung (5) abschnittsweise ausgenommen ist, wobei der zweite Fluidkanal (8) durch die zweite Nut (17) und durch die zweite Gehäusenut (18) gebildet ist.

8. Medizinische Fluidsteuervorrichtung (1) nach einem der vorhergehenden Ansprüche, **dadurch gekennzeichnet, dass** in dem ersten Fluidkanal (7) ein Widerstandselement (20) oder/und eine Engstelle (19) angeordnet ist; und/oder dass in dem zweiten Fluidkanal (8) ein Widerstandselement (20) oder/und eine Engstelle (19) angeordnet ist.

9. Medizinische Fluidsteuervorrichtung (1) nach Anspruch 8, **dadurch gekennzeichnet, dass** das Widerstandselement (20) einen Grundkörper (21) umfasst, welcher ein fluiddurchlässiges poröses Material (22), insbesondere ein Sintermaterial, umfasst oder aus einem solchen Material (22) besteht, oder/und wenigstens eine beidenends geöffnete fluiddurchlässige Kapillare (23) aufweist, oder/und eine Außenfläche (24) aufweist, zwischen welcher und einer den jeweiligen Fluidkanal (7, 8) begrenzenden Kanalwand (25) wenigstens ein fluiddurchlässiger Zwischenraum ausgebildet ist, wobei vorzugsweise an der Außenfläche (24) eine rückspringende Rille (27) zur Ausbildung des fluiddurchlässigen Zwischenraums vorhanden ist.
